# EUROPEAN PATENT APPLICATION

(11) **EP 3 825 311 A1**
(43) Date of publication of application: **26.05.2021**
(21) Application number: 19837834.1
(22) Date of filing: 18.07.2019
(51) Int. Cl.: C07D 401/12, C07D 401/14, C07D 405/14, A61P 9/10, A61P 11/00

(54) **METHOD FOR PREPARING COAGULATION FACTOR XIA INHIBITOR AND INTERMEDIATE THEREOF**

(30) Priority: 19.07.2018 CN 201810796646
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: XI, Zhuoxun, Shanghai 200245 (CN); FENG, Yingqiang, Shanghai 200245 (CN); FENG, Jun, Shanghai 200245 (CN); HE, Feng, Shanghai 200245 (CN); HUANG, Jian, Lianyungang, Jiangsu 222047 (CN); MAO, Yanli, Lianyungang, Jiangsu 222047 (CN); WANG, Yong, Lianyungang, Jiangsu 222047 (CN); GUAN, Zhongjun, Lianyungang, Jiangsu 222047 (CN)
(74) Representative: Potter Clarkson
(86) International application number: PCT/CN2019/096504
(87) International publication number: WO 2020/015698

(57) **Abstract**

The present disclosure involves a method for preparing a coagulation factor XIa inhibitor and an intermediate thereof. Specifically, the present disclosure involves a method for preparing an oxypyridine amide derivative. The method has the advantages of high yield, good product purity, and mild reaction conditions.

## Description

### FIELD OF THE INVENTION

The present disclosure belongs to the field of medicine, and relates to a method for preparing a blood coagulation factor XIa inhibitor.

### BACKGROUND OF THE INVENTION

The process of human blood coagulation, consisting of an intrinsic pathway, an extrinsic pathway, and a common pathway, is a chain reaction in which the process is continuously enhanced and amplified by sequential activation of multiple zymogens. The coagulation cascade is initiated by the endogenous pathway (also known as the contact activation pathway) and the exogenous pathway (also known as the tissue factor pathway) to produce FXa, which then forms thrombin (FIIa) by the common pathway, and fibrin is finally formed.

The endogenous pathway refers to the process from the activation of factor XII to the formation of XIa-VIIIa-Ca2±P L complex and the activation of factor X, and the exogenous blood coagulation pathway refers to the process from the release of tissue factor (TF) to the formation of TF-VIIaCa2+ complex and the activation of factor X. The common pathway refers to the process in which after the formation of factor Xa, the two pathways are combined into one, prothrombin is activated, and fibrin is finally formed, wherein FXI is necessary for maintaining the endogenous pathway and plays a key role in the amplification of the blood coagulation cascade. In the blood coagulation cascade, thrombin can feedback activate FXI, and activated FXI (FXIa) in turn promotes the mass production of thrombin, thereby amplifying the blood coagulation cascade. Therefore, antagonists of FXI have been extensively developed for the treatment of various thrombi.

Patent application WO2018041122 discloses a class of effective FXIa inhibitors and a method for preparing the same. Among them, the compound of formula I and the compound of formula VI have obvious pharmacodynamic advantages. The existing preparation method is to prepare a racemate, which is then resolved to obtain the target compound.

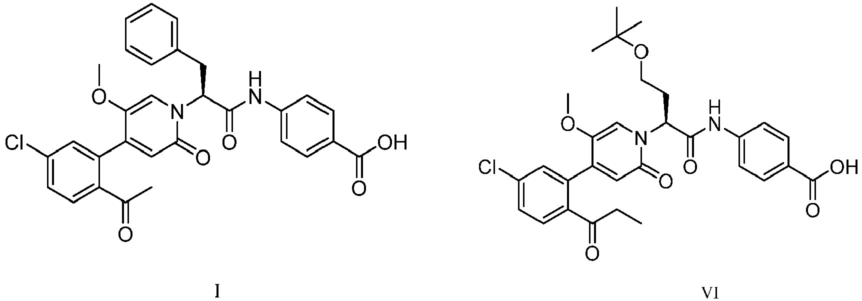

### SUMMARY OF THE INVENTION

The object of the present disclosure is to provide a novel method for preparing a blood coagulation factor XIa inhibitor.

In an aspect, the present disclosure provides a method for preparing a compound of formula AI, comprising a step of reacting a compound of formula III and a compound of formula II, 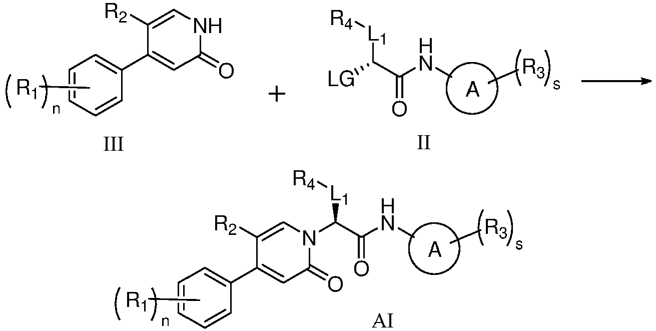 wherein:
ring A is an aryl or heteroaryl;
each R₁ is identical or different and each is independently selected from the group consisting of hydrogen atom, halogen, alkyl, haloalkyl, alkoxy, haloalkoxy, amino, nitro, cyano, hydroxy, hydroxyalkyl, -C(O)R₅, cycloalkyl, heterocyclyl, aryl and heteroaryl;
R₂ is selected from the group consisting of halogen, alkyl, haloalkyl, alkoxy, cycloalkyloxy, haloalkoxy, amino, nitro, cyano, hydroxy, hydroxyalkyl and alkylthio, wherein the alkyl, alkoxy, haloalkyl, haloalkoxy, hydroxyalkyl and alkylthio are each optionally further substituted by one or more substituent(s) selected from the group consisting of deuterium atom, halogen, alkoxy, haloalkoxy, amino, nitro, cyano, hydroxy and hydroxyalkyl;
L₁ is an alkylene, wherein the alkylene is optionally further substituted by one or more substituent(s) selected from the group consisting of halogen and deuterium atom;
each R₃ is identical or different and each is independently selected from the group consisting of hydrogen atom, alkyl, alkoxy, oxo, halogen, haloalkyl, haloalkoxy, amino, nitro, cyano, hydroxy, hydroxyalkyl, cycloalkyl, heterocyclyl, aryl, heteroaryl, -C(O)R₆, -C(O)OR₆, -NHC(O)R₆, -NHC(O)OR₆, -NR₇R₈, -C(O)NR₇R₈, -CH₂NHC(O)OR₆, -CH₂NR₇R₈, -C(O)OCH(R₇)R₈ and -S(O)ₘR₆, wherein the alkyl is optionally further substituted by one or more substituent(s) selected from the group consisting of deuterium atom, halogen, alkoxy, haloalkoxy, amino, nitro, cyano, hydroxy, hydroxyalkyl, -NR₇R₈ and -NHC(O)OR₆;
R₄ is selected from the group consisting of hydrogen atom, deuterium atom, halogen, alkyl, alkoxy, amino, nitro, cyano, hydroxy, hydroxyalkyl, cycloalkyl, cycloalkyloxy, heterocyclyl, aryl and heteroaryl, wherein the cycloalkyl, heterocyclyl, aryl and heteroaryl are each optionally further substituted by one or more substituent(s) selected from the group consisting of deuterium atom, halogen, alkyl, alkoxy, haloalkyl, amino, nitro, cyano, hydroxy, hydroxyalkyl, cycloalkyl, cycloalkyloxy, heterocyclyl, -NHC(O)R₉ and R₁₀;
R₅ is selected from the group consisting of hydrogen atom, alkyl, haloalkyl, alkoxy, haloalkoxy, amino, hydroxy, hydroxyalkyl, cycloalkyl, heterocyclyl, aryl and heteroaryl, wherein the alkyl, alkoxy, cycloalkyl, heterocyclyl, aryl and heteroaryl are each optionally further substituted by one or more substituent(s) selected from the group consisting of hydrogen atom, deuterium atom, halogen, alkyl, alkoxy, haloalkoxy, amino, nitro, cyano, hydroxy, hydroxyalkyl, cycloalkyl, cycloalkyloxy, heterocyclyl, aryl and heteroaryl;
R₆ is selected from the group consisting of hydrogen atom, alkyl, haloalkyl, hydroxy, amino, cycloalkyl, heterocyclyl, aryl, heteroaryl and carboxy protecting group;
R₇ and R₈ are identical or different and are each independently selected from the group consisting of hydrogen atom, alkyl, haloalkyl, cycloalkyl, heterocyclyl, -C(O)OR₆ and -OC(O)OR₉, wherein the cycloalkyl and heterocyclyl are each optionally further substituted by one or more substituent(s) selected from the group consisting of deuterium atom, halogen, alkyl, alkoxy, haloalkyl, oxo, amino, nitro, cyano, hydroxy and hydroxyalkyl;
R₉ is selected from the group consisting of hydrogen atom, alkyl, alkoxy, haloalkyl, haloalkoxy, amino, cycloalkyl, heterocyclyl, aryl and heteroaryl;
R₁₀ is an aryl or heteroaryl, wherein the aryl and heteroaryl are each optionally further substituted by one or more substituent(s) selected from the group consisting of hydrogen atom, deuterium atom, halogen, alkyl, alkoxy, haloalkyl, amino, nitro, cyano, hydroxy, hydroxyalkyl, cycloalkyl, cycloalkyloxy and heterocyclyl;
LG is a leaving group, and preferably a halogen, substituted sulfonyloxy, RᵢRⱼN-, hydroxy, cyano, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
n is 0, 1, 2, 3 or 4;
m is 0, 1 or 2; and
s is 0, 1, 2, 3 or 4.

In some embodiments, the compound of formula III reacts with the compound of formula II in the presence of an alkaline substance.

The alkaline substance can be one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)₂, LiORₐ, NaORₐ, KORₐ, CsORₐ, T1ORₐ, NR_{b}R_{c}R_{d}, DBU, DBN, Mg(ORₐ)₂, LiRₑ, NaNH₂, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, and X is a halogen. The alkaline substance is preferably one or more of tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ and Na₃PO₄.

The solvent used in the reaction can be a conventional solvent, for example one or more of water, dimethylformamide, 1-methyl-2-pyrrolidone, tetrahydrofuran, methyltetrahydrofuran, dioxane, toluene, xylene, dimethyl sulfoxide, diethyl ether, isopropyl ether, methyl *tert-butyl* ether, acetonitrile, propionitrile, C₁-C₆ alkyl alcohol, acetone and ethyl acetate.

The reaction temperature of the reaction can be -20°C to 100°C, preferably -20°C to 60°C, and more preferably -10°C to 40°C.

In some embodiments, the molar ratio of the compound of formula II to the alkaline substance can be 1:1 to 1:20, and preferably 1:1 to 1:8.

In some embodiments, the substituted sulfonyloxy can be a C₁-C₆ alkylsulfonyloxy, perfluoro C₁-C₆ alkylsulfonyloxy, arylsulfonyloxy, aralkylsulfonyloxy or heteroarylsulfonyloxy.

Specific examples of C₁-C₆ alkylsulfonyloxy include linear or branched C₁-C₆ alkylsulfonyloxy, for example methylsulfonyloxy, ethylsulfonyloxy, *n*-propylsulfonyloxy, isopropylsulfonyloxy, *n*-butylsulfonyloxy, *tert*-butylsulfonyloxy, *n*-pentylsulfonyloxy or *n*-hexylsulfonyloxy.

Specific examples of perfluoro C₁-C₆ alkylsulfonyloxy include linear or branched perfluoro C₁-C₆ alkylsulfonyloxy, for example trifluoromethylsulfonyloxy, 1,1,2,2,2-pentafluoro-1-ethylsulfonyloxy, 1,1,2,2,3,3,3 -heptafluoro-1 -propylsulfonyloxy or 1,1,2,2,3,3,4,4,4-nonafluoro-1-butylsulfonyloxy.

Examples of arylsulfonyloxy include phenylsulfonyloxy and naphthylsulfonyloxy in which the benzene ring optionally has one to three substituent(s) selected from the group consisting of linear or branched C₁-C₆ alkyl, linear or branched C₁-C₆ alkyl, nitro and halogen atom. Specific examples of optionally substituted phenylsulfonyloxy include phenylsulfonyloxy, 4-methylphenylsulfonyloxy, 2-methylphenylsulfonyloxy, 4-nitrophenylsulfonyloxy, 4-tolylsulfonyloxy, 2-nitrophenylsulfonyloxy, 3-chlorophenylsulfonyloxy and the like. Specific examples of naphthylsulfonyloxy include α-naphthylsulfonyloxy, β-naphthylsulfonyloxy and the like.

Examples of aralkylsulfonyloxy include linear or branched C₁-C₆ alkylsulfonyloxy substituted by phenyl (in which the benzene ring optionally has one to three substituent(s) selected from the group consisting of linear or branched C₁-C₆ alkyl, linear or branched C₁-C₆ alkyl, nitro and halogen atom) and linear or branched C₁-C₆ alkylsulfonyloxy substituted by naphthyl. Specific examples of alkylsulfonyloxy substituted by phenyl include benzylsulfonyloxy, 2-phenylethylsulfonyloxy, 4-phenylbutylsulfonyloxy, 4-methylbenzylsulfonyloxy, 2-methylbenzylsulfonyloxy, 4-nitrobenzylsulfonyloxy, 4-methylbenzylsulfonyloxy, 3-chlorobenzylsulfonyloxy and the like. Specific examples of alkylsulfonyloxy substituted by naphthyl include α-naphthylmethylsulfonyloxy, β-naphthylmethylsulfonyloxy and the like.

Examples of substituted phosphoryloxy and substituted formyloxy are basically the same as the examples of substituted sulfonyloxy.

In some embodiments, the leaving group can be p-toluenesulfonyloxy, trifluoromethanesulfonyloxy, methanesulfonyloxy, p-nitrobenzenesulfonyloxy and the like.

In some embodiments, the compound of formula II is a compound of formula IIa, 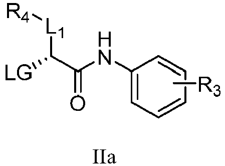 wherein L₁, R₃, R₄ and LG are as defined above.

In some embodiments, the compound of formula II is a compound of formula lib, 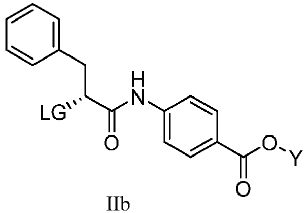 wherein LG is a leaving group, preferably a halogen, substituted sulfonyloxy, RᵢRⱼN-, hydroxy, cyano, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, cyano, RₖS-, substituted or unsubstituted phosphoryloxy or substituted formyloxy; and
Y is selected from the group consisting of hydrogen atom and carboxy protecting group, and preferably a hydrogen atom, methyl, ethyl, allyl, isopentenyl and trimethylsilylethyl.

In some embodiments, the compound of formula II is a compound of formula IIc, 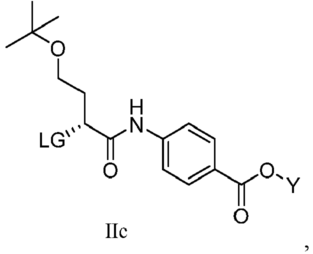 wherein LG is a leaving group, preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS-, substituted or unsubstituted phosphoryloxy or substituted formyloxy; and
Y is selected from the group consisting of hydrogen atom and carboxy protecting group, and preferably a hydrogen atom, methyl, ethyl, allyl, isopentenyl and trimethylsilylethyl.

In some embodiments, the compound of formula III is a compound of formula IIIa, 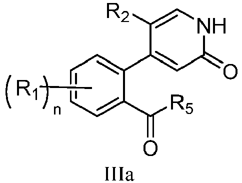 wherein each R₁ is identical or different and each is independently selected from the group consisting of hydrogen atom, halogen, alkyl, haloalkyl, alkoxy, haloalkoxy, amino, nitro, cyano, hydroxy, hydroxyalkyl, cycloalkyl, heterocyclyl, aryl and heteroaryl;
R₅ is selected from the group consisting of alkyl, cycloalkyl and haloalkyl, wherein the alkyl and cycloalkyl are each optionally further substituted by one or more substituent(s) selected from the group consisting of deuterium atom, halogen, alkyl and cycloalkyl; and
R₂ and n are as defined above.

In some embodiments, the compound of formula III is a compound of formula IIIb,

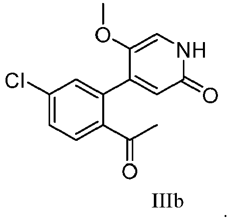

In some embodiments, the compound of formula III is a compound of formula IIIc,

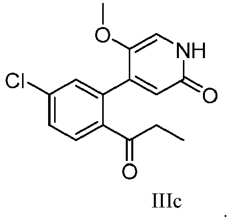

In some embodiments, the method also comprises a step of reacting a compound of formula IV and a compound of formula V to obtain the compound of formula II, 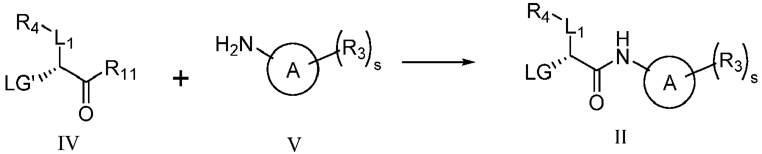 wherein R₁₁ is selected from the group consisting of -OR₉ and halogen;
LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and
L₁, R₃, R₄, R₉, LG and s are as defined above.

In another aspect, the present disclosure provides a compound of formula IIb, 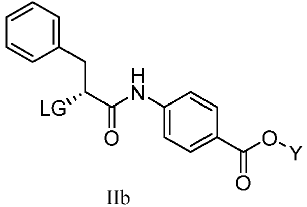 wherein LG is a leaving group, preferably a halogen, substituted sulfonyloxy, RᵢRⱼN-, hydroxy, cyano, RₖS- or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, cyano, RₖS- or substituted formyloxy; and
Y is selected from the group consisting of hydrogen atom and carboxy protecting group, and preferably a hydrogen atom, methyl, ethyl, allyl, isopentenyl and trimethyl silyl ethyl;
and when LG is a hydroxy, Y is not a methyl.

In another aspect, the present disclosure provides a method for preparing the compound of formula lib, comprising a step of reacting a compound of formula IVb or a salt thereof and a compound of formula Vb or a salt thereof, 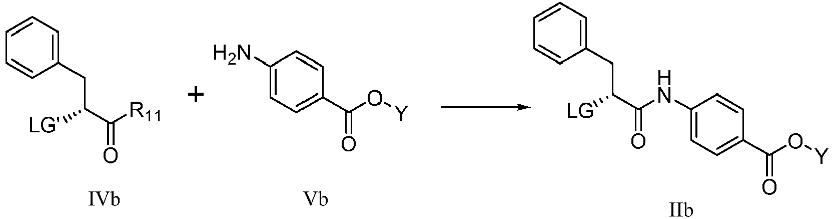 wherein R₁₁ is selected from the group consisting of -OR₉ and halogen; R₉ is selected from the group consisting of hydrogen atom, alkyl, alkoxy, haloalkyl, haloalkoxy, amino, cycloalkyl, heterocyclyl, aryl and heteroaryl; LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and LG and Y are as defined in the compound of formula lib.

In some embodiments, the salt of the compound of formula IVb can be a sodium salt, potassium salt, amine salt and the like.

In some embodiments, the salt of the compound of formula Vb can be a hydrochloride, sulfate, phosphate, acetate and the like.

In another aspect, the present disclosure provides a compound of formula IIc, 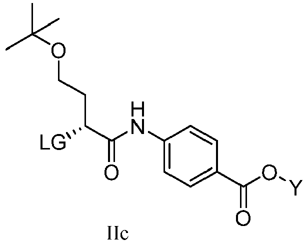 wherein LG is a leaving group, preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS- or substituted formyloxy; and
Y is selected from the group consisting of hydrogen atom and carboxy protecting group, and preferably a hydrogen atom, methyl, ethyl, allyl, isopentenyl and trimethylsilylethyl.

In another aspect, the present disclosure provides a method for preparing the compound of formula Iic, comprising a step of reacting a compound of formula IVc or a salt thereof and a compound of formula Vb or a salt thereof, 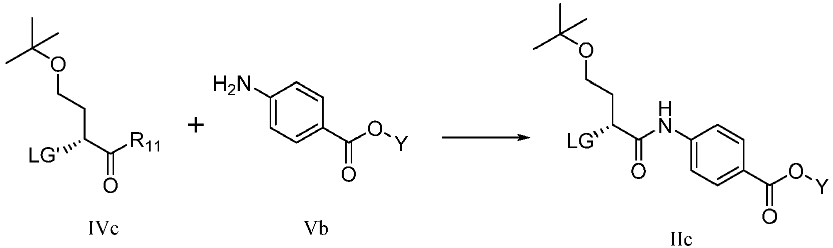 wherein R₁₁ is selected from the group consisting of -OR₉ and halogen; R₉ is selected from the group consisting of hydrogen atom, alkyl, alkoxy, haloalkyl, haloalkoxy, amino, cycloalkyl, heterocyclyl, aryl and heteroaryl; LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and LG and Y are as defined in the compound of formula IIc.

In some embodiments, the salt of the compound of formula IVc can be a sodium salt, potassium salt, amine salt and the like.

In some embodiments, the salt of the compound of formula Vb can be a hydrochloride, sulfate, phosphate, acetate and the like.

In another aspect, the present disclosure also provides a compound of formula IVd, 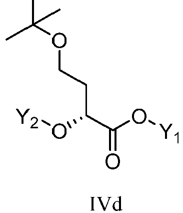 wherein Y1 is selected from the group consisting of hydrogen atom and carboxy protecting group; and Y2 is selected from the group consisting of hydrogen atom and hydroxy protecting group.

In another aspect, the present disclosure also provides a method for preparing the compound of formula Ivd, comprising a step of converting a compound of formula IVe into the compound of formula IVd,

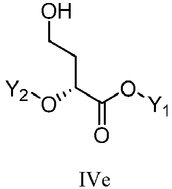

In another aspect, the present disclosure also provides a method for preparing the compound of formula I or the pharmaceutically acceptable salt thereof, comprising a step of reacting the compound of formula IIIb and the compound of formula IIb to obtain a compound of formula BI, wherein LG and Y are as defined above,

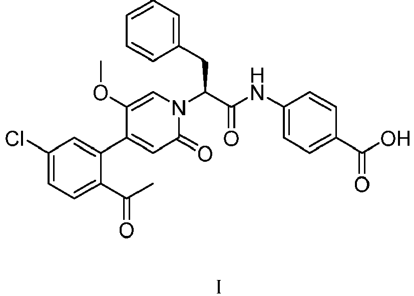

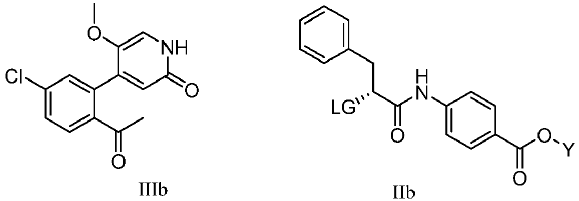

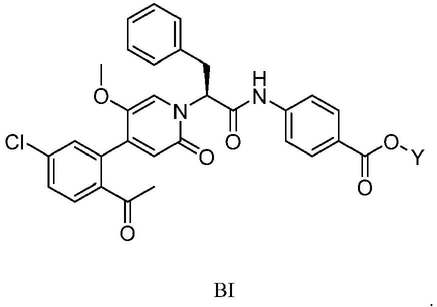

In some embodiments, the compound of formula IIIb reacts with the compound of formula IIb in the presence of an alkaline substance.

The alkaline substance can be one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)₂, LiORₐ, NaORₐ, KORₐ, CsORₐ, T1ORₐ, NR_{b}R_{c}R_{d}, DBU, DBN, Mg(ORₐ)₂, LiRₑ, NaNH₂, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, and X is a halogen. The alkaline substance is preferably tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ or Na₃PO₄.

The solvent used in the reaction can be a conventional solvent, for example one or more of water, dimethylformamide, 1-methyl-2-pyrrolidone, tetrahydrofuran, methyltetrahydrofuran, dioxane, toluene, xylene, dimethyl sulfoxide, diethyl ether, isopropyl ether, methyl *tert-butyl* ether, acetonitrile, propionitrile, C₁-C₆ alkyl alcohol, acetone and ethyl acetate.

The reaction temperature of the reaction can be -20°C to 100°C, preferably -20°C to 60°C, and more preferably -10°C to 40°C.

In some embodiments, the molar ratio of the compound of formula IIb to the alkaline substance can be 1:1 to 1:20, and preferably 1:1 to 1:8.

In some embodiments, the method can also comprise the step for preparing the compound of formula IIb according to the present disclosure.

In some embodiments, LG is a substituted sulfonyloxy, and the method also comprises a step of converting a compound of formula IIcIId into the compound of formula IIb,

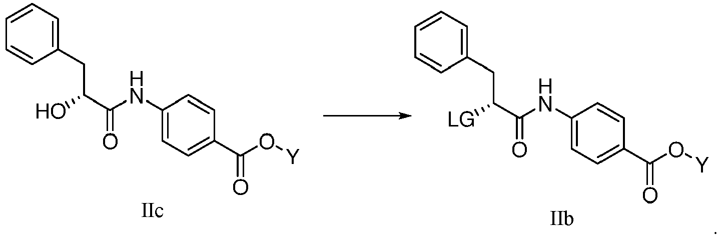

The compound of formula IIcIId can be prepared by the method for preparing the compound of formula II according to the present disclosure.

In some embodiments, when Y is a carboxy protecting group, the method can also comprise a step of removing the carboxy protecting group of the compound of formula BI.

In another aspect, the present disclosure also provides a method for preparing the compound of formula VI or the pharmaceutically acceptable salt thereof, comprising a step of reacting the compound of formula IIIc and the compound of formula IIc to obtain a compound of formula BVI, wherein LG and Y are as defined above,

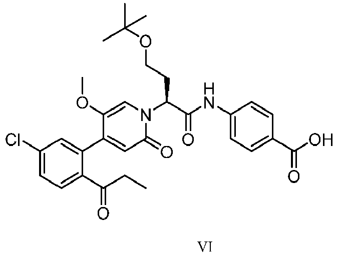

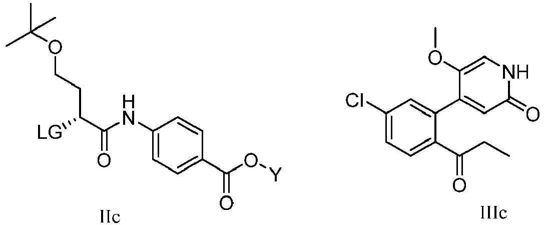

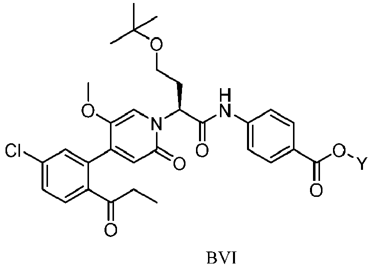

In some embodiments, the compound of formula IIIc reacts with the compound of formula IIc in the presence of an alkaline substance.

The alkaline substance can be one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)₂, LiORₐ, NaORₐ, KORₐ, CsORₐ, T1ORₐ, NR_{b}R_{c}R_{d}, DBU, DBN, Mg(ORₐ)₂, LiRₑ, NaNH2, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, and X is a halogen. The alkaline substance is preferably tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ or Na₃PO₄.

The solvent used in the reaction can be a conventional solvent, for example one or more of water, dimethylformamide, 1-methyl-2-pyrrolidone, tetrahydrofuran, methyltetrahydrofuran, dioxane, toluene, xylene, dimethyl sulfoxide, diethyl ether, isopropyl ether, methyl *tert-butyl* ether, acetonitrile, propionitrile, C₁-C₆ alkyl alcohol, acetone and ethyl acetate.

The reaction temperature of the reaction can be -20°C to 100°C, preferably -20°C to 60°C, and more preferably -10°C to 40°C.

In some embodiments, the molar ratio of the compound of formula IIc to the alkaline substance can be 1:1 to 1:20, and preferably 1:1 to 1:8.

In some embodiments, the method can also comprise the step for preparing the compound of formula IIc according to the present disclosure.

In some embodiments, LG is a substituted sulfonyloxy, and the method also comprises a step of converting a compound of formula IIc into the compound of formula IIc,

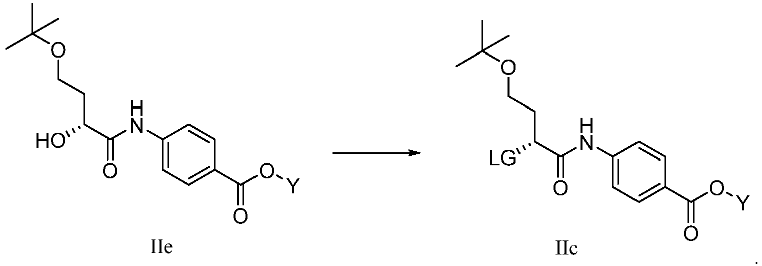

The compound of formula IIc can be prepared by the method for preparing the compound of formula II according to the present disclosure.

In some embodiments, when Y is a carboxy protecting group, the method can also comprise a step of removing the carboxy protecting group of the compound of formula BVI.

The method for preparing the FXIa inhibitor and intermediates thereof according to the present disclosure directly provides the stereoisomeric target product by chiral synthesis without the need to resolve the racemate. The optical purity of the product during the entire reaction process is maintained, and the reaction yield is greatly improved, thereby facilitating industrial production.

Unless otherwise stated, the terms used in the specification and claims have the meanings described below.

The term "alkyl" refers to a saturated aliphatic hydrocarbon group, which is a straight or branched chain group comprising 1 to 20 carbon atoms, and preferably an alkyl having 1 to 12 carbon atoms. Non-limiting examples include methyl, ethyl, *n*-propyl, isopropyl, n-butyl, isobutyl, *tert-butyl,* sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, *tert-butyl,* sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkylene" refers to a saturated linear or branched aliphatic hydrocarbon group having two residues derived from the removal of two hydrogen atoms from the same carbon atom or two different carbon atoms of the parent alkane. The alkylene is a linear or branched alkylene having 1 to 20 carbon atoms, preferably 1 to 12 carbon atoms, and more preferably 1 to 6 carbon atoms. Non-limiting examples of alkylene include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-) and the like. The alkylene can be substituted or unsubstituted. When substituted, the substituent group(s) can be substituted at any available connection point.

The term "cycloalkyl" refers to a saturated or partially unsaturated monocyclic or polycyclic hydrocarbon substituent having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "heterocyclyl" refers to a 3 to 20 membered saturated or partially unsaturated monocyclic or polycyclic hydrocarbon substituent, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; and more preferably, 3 to 6 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably piperidinyl and pyrrolidinyl. Polycyclic heterocyclyl includes a heterocyclyl having a spiro ring, fused ring or bridged ring.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl ring can be fused to the ring of heteroaryl, heterocyclyl or cycloalkyl, wherein the ring bound to the parent structure is aryl ring. Non-limiting examples thereof include: 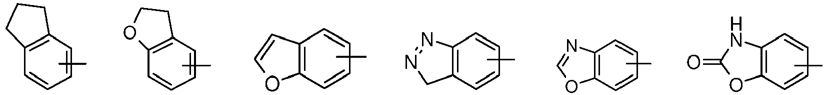 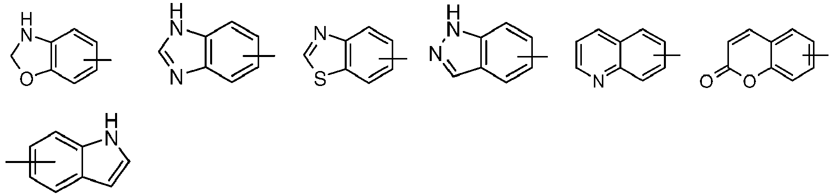 and

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl, and preferably phenyl.

The term "heteroaryl" refers to a 5 to 14 membered heteroaromatic system having 1 to 4 heteroatoms selected from the group consisting of O, S and N. The heteroaryl is preferably a 5 to 12 membered heteroaryl, for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, pyrazolyl, pyrimidinyl and thiazolyl, and more preferably pyrazolyl and thiazolyl. The heteroaryl ring can be fused to the ring of aryl, heterocyclyl or cycloalkyl, wherein the ring bound to the parent structure is heteroaryl ring. Non-limiting examples thereof include:

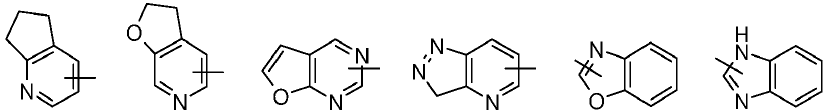

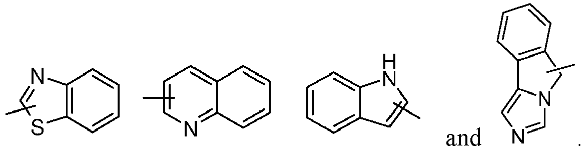

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkylthio" refers to an -S-(alkyl) or an -S-(unsubstituted cycloalkyl) group, wherein the alkyl is as defined above. Non-limiting examples of alkylthio include methylthio, ethylthio, propylthio, butylthio, cyclopropylthio, cyclobutylthio, cyclopentylthio, cyclohexylthio. The alkylthio can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "halogen" refers to fluorine, chlorine, bromine or iodine.

"Carboxy protecting group" is a group known in the art suitable for protecting carboxy, see carboxy protecting groups disclosed in the document ("Protective Groups in Organic Synthesis", 5^{Th} Ed. T. W. Greene & P. G M. Wuts). As an example, preferably, the carboxy protecting group can be a substituted or unsubstituted linear or branched C₁₋₁₀ alkyl, substituted or unsubstituted linear or branched C₂₋₁₀ alkenyl or alkynyl, substituted or unsubstituted C₃₋₈ cycloalkyl, substituted or unsubstituted C₅₋₁₀ aryl or heteroaryl, or (C₁₋₈ alkyl or aryl)₃silyl, preferably a linear or branched C₁₋₆ alkyl, and more preferably a linear or branched C₁₋₄ alkyl. For example, the carboxy protecting group can be a methyl, ethyl, allyl, isopentenyl, trimethylsilylethyl and the like.

"Amino protecting group" is a group known in the art suitable for protecting amino, see amino protecting groups disclosed in the document ("Protective Groups in Organic Synthesis", 5Th Ed. T. W. Greene & P. G M. Wuts). Preferably, the amino protecting group can be a (C₁₋₁₀ alkyl or aryl)acyl, for example, formyl, acetyl, benzoyl and the like; (C₁₋₆ alkyl or C₆₋₁₀ aryl)sulfonyl; (C₁₋₆ alkoxy or C₆₋₁₀ aryloxy)carbonyl, Boc or Cbz; and alkyl, for example, trityl (Tr), 2,4-dimethoxybenzyl (DMB), p-methoxybenzyl (PMB) or benzyl (Bn).

"Hydroxy protecting group" is a group known in the art suitable for protecting hydroxy, see hydroxy protecting groups disclosed in the document ("Protective Groups in Organic Synthesis", 5Th Ed. T. W. Greene & P. G M. Wuts). As an example, preferably, the hydroxy protecting group can be a (C₁₋₁₀ alkyl or aryl)₃silyl, for example, triethylsilyl, triisopropylsilyl, *tert*-butyldimethylsilyl, *ter*t-butyldiphenylsilyl and the like; C₁₋₁₀ alkyl or substituted alkyl, preferably an alkyl substituted by alkoxy or aryl, more preferably a C₁₋₆ alkyl substituted by C₁₋₆ alkoxy or C₁₋₆ alkyl substituted by phenyl, and most preferably a C₁₋₄ alkyl substituted by C₁₋₄ alkoxy, for example, methyl, *tert-butyl,* allyl, benzyl, methoxymethyl (MOM), ethoxyethyl, 2-tetrahydropyranyl (THP) and the like; (C₁₋₁₀ alkyl or aryl)acyl, for example, formyl, acetyl, benzoyl and the like; (C₁₋₆ alkyl or C₆₋₁₀ aryl)sulfonyl; and (C₁₋₆ alkoxy or C₆₋₁₀ aryloxy)carbonyl.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

In the chemical structure of the compound of the present disclosure, the bond " 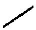 " does not specify a configuration, that is, if there is configurational isomerism in the chemical structure, the bond " 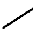 " can be " 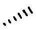 " or " 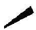 ", or it can contain both " 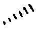 " and " 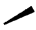 " configurations.

### DETAILED DESCRIPTION OF THE INVENTION

The present disclosure will be explained in detail below in conjunction with the specific examples, allowing those skilled in the art to understand the disclosure comprehensively. The specific examples are only used to illustrate the technical solution of the present disclosure, and will not limit the present disclosure in any way.

### Example 1

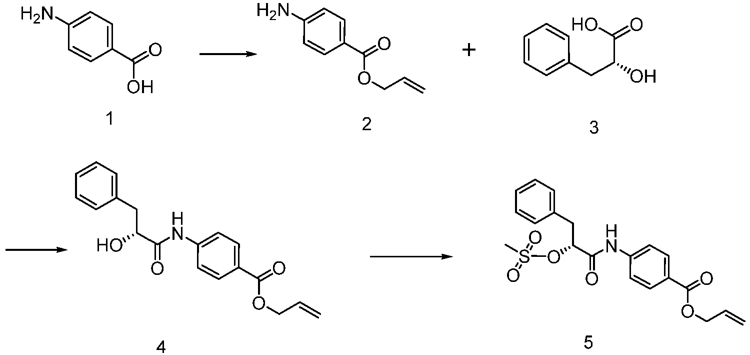

### Step 1

Compound 1 (40.1 g, 292.41 mmol) was dissolved in 500 mL of DMF, followed by the addition of potassium carbonate (40.41 g, 292.41 mmol). The reaction solution was cooled in an ice bath, followed by the addition of propenyl bromide (35.37 g, 292.41 mmol). The reaction solution was reacted at room temperature. After completion of the reaction, 1500 mL of water was added to the reaction solution, and the resulting solution was extracted with ethyl acetate. The organic phases were combined, washed with saturated sodium chloride solution and water successively, dried over anhydrous sodium sulfate, and filtered. The filtrate was concentrated under reduced pressure, and the resulting residue was purified by silica gel column chromatography (eluent: *n*-hexane: ethyl acetate= 10:1). The product was collected and concentrated to obtain compound 2 (33.7g, yield: 65%).
MS m/z (LC-MS): 178.2[M+1]

### Step 2

Compound 2 (532 mg, 3 mmol), D-phenyllactic acid (compound 3, 500 mg, 3 mmol) and methylboronic acid (18 mg, 0.3 mmol) were added to a reaction flask, followed by the addition of 20 mL of toluene and 108 µL of water. The reaction solution was warmed up to 140°C, and reacted under reflux. After completion of the reaction, the reaction solution was cooled to room temperature, followed by the addition of 100 mL of dichloromethane. The solution was washed with 0.5 M hydrochloric acid solution and 1 M sodium bicarbonate solution successively, dried over anhydrous sodium sulfate, and filtered. The filtrate was concentrated under reduced pressure, and the resulting residue was purified by Combiflash (eluent: 100% dichloromethane) to obtain compound 4 (829 mg, yield: 84.9%).
MS m/z (LC-MS): 326.2[M+1]

### Step 3

Compound 4 (4.3 g, 13.22 mmol) was dissolved in 50 mL of tetrahydrofuran. The resulting solution was purged with argon, cooled in an ice bath, followed by the addition of triethylamine (2.67 g, 26.43 mmol) and the dropwise addition of methylsulfonyl chloride (2.27 mg, 19.82 mmol). The reaction solution was reacted in an ice bath. After completion of the reaction, 1 M dilute hydrochloric acid solution was added to the reaction solution to quench the reaction. Two phases were separated, and the aqueous phase was extracted with ethyl acetate. The organic phases were combined, washed with saturated aqueous sodium chloride solution, dried over anhydrous sodium sulfate, and filtered. The filtrate was concentrated, and the resulting crude product was pulped in methyl *tert-butyl* ether, filtered, and dried to obtain compound 5 (4.6 g, yield: 86.27%).
MS m/z (LC-MS): 404.1[M+1]

### Example 2

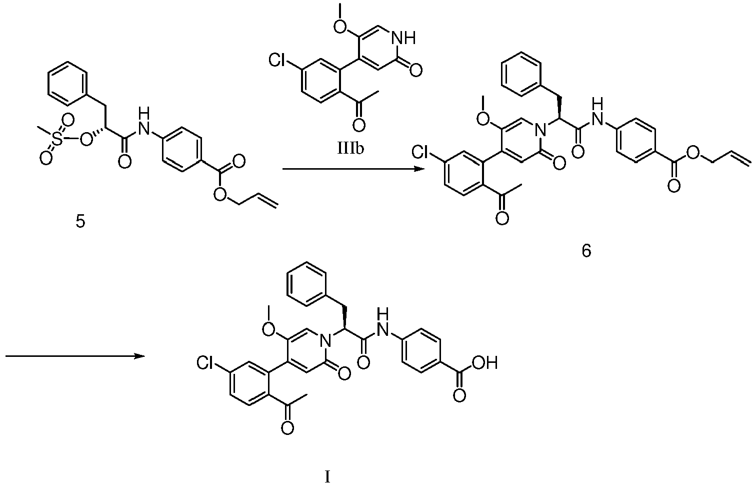

### Step 1

Compound IIIb (6.54 g, 23.55 mmol, prepared according to the method disclosed in the patent application WO2018041122) was dissolved in 200 mL of tetrahydrofuran, followed by the addition of tetrabutylammonium iodide (7.84 g, 23.55 mmol) and potassium *tert*-butoxide (3.96 g, 35.32 mmol). Under an argon atmosphere, the solution was cooled to 0°C, followed by the addition of compound 5 (9.5 g, 23.55 mmol). The reaction solution was kept at this temperature and reacted. After completion of the reaction, 1 M hydrochloric acid solution was added to the reaction solution to quench the reaction, followed by the addition of 50 ml of ethyl acetate and 100 ml of water under stirring. Two phases were separated, and the aqueous phase was extracted with ethyl acetate. The organic phases were combined, washed with saturated sodium chloride solution, dried over anhydrous sodium sulfate, and filtered. The filtrate was concentrated under reduced pressure, and the resulting residue was purified by silica gel column chromatography (eluent: *n*-hexane: ethyl acetate: dichloromethane= 10:3:1) to obtain compound 6 (8.1 g, yield: 58.8%, optical purity: 95.7%).

### Step 2

Compound 6 (8.10 g, 13.85 mmol) was dissolved in 81 mL of tetrahydrofuran, and the solution was cooled to 0°C under an argon atmosphere. Pyrrolidine (984.67 mg, 13.85 mmol) and tetrakis(triphenylphosphine) palladium (1.60 g, 1.38 mmol) were added, and the reaction solution was stirred at 0°C. After completion of the reaction, 1 M hydrochloric acid solution was added to the reaction solution to quench the reaction. 40 ml of water was added, and the solution was stirred and filtered. Two phases were separated, and the aqueous phase was extracted with ethyl acetate. The organic phases were combined, washed with saturated sodium chloride solution, dried over anhydrous sodium sulfate, and filtered. The filtrate was concentrated under reduced pressure, and 80 mL of methanol was added to the resulting residue. The solution was warmed up to 50°C, and pulped for 30 minutes. The solution was naturally cooled to room temperature, stirred for 30 minutes, and filtered. The filter cake was collected, and dried to obtain the compound of formula I (6.48 g, yield: 85.88%, optical purity: 93.8%).

### Example 3

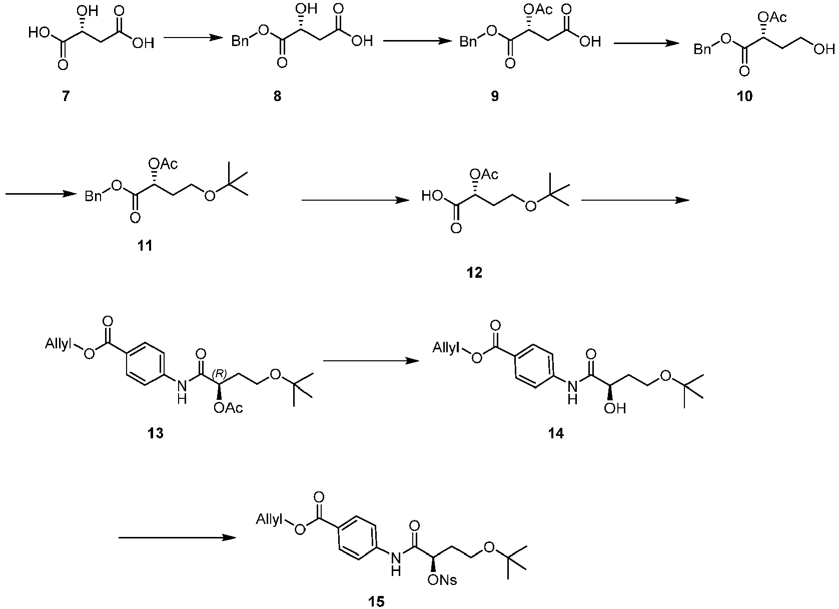

### Step 1

40 g of (*R*)-malic acid was added to a reaction flask, followed by the addition of 250 g of trifluoroacetic anhydride. The reaction solution was reacted at room temperature for 2 hours, followed by the removal of solvent by evaporation. BnOH (167 mL) was added, and the reaction solution was stirred at room temperature. After completion of the reaction, sodium bicarbonate solution was added to the system, which was then extracted with ethyl acetate. The aqueous phase was adjusted to be acidic with hydrochloric acid, and extracted with ethyl acetate. The organic phases were collected, combined, and concentrated to obtain compound 8 (69 g).

### Step 2

Compound 8 (69 g) was added to a reaction flask, followed by the addition of 65.1 g of acetic anhydride and 490 mg of anhydrous ferric chloride. The reaction solution was reacted at room temperature. After completion of the reaction, acetonitrile was added to the reaction solution. The solution was added to saturated sodium bicarbonate solution, stirred, and extracted with ethyl acetate. The aqueous phase was adjusted to be acidic with hydrochloric acid, and extracted with ethyl acetate. The organic phases were collected, combined, and concentrated to obtain compound 9 (56.5 g, yield: 70%).

### Step 3

Compound 9 (56.5 g) was added to a reaction flask, followed by the addition of 200 mL of tetrahydrofuran. The solution was cooled to 0°C, followed by the dropwise addition of 10.0 M borane dimethyl sulfide solution (42 mL). After completion of the addition, the reaction solution was stirred at room temperature. After completion of the reaction, methanol was added to quench the reaction. The system was concentrated to obtain compound 10 (49.4g, yield: 92%).

### Step 4

Compound 10 (29 g), *tert-butyl* trichloroacetimide (37.6 g) and dichloromethane (230 mL) were added to a reaction flask, and stirred to dissolve. The solution was cooled to -40°C, followed by the addition of TBSOTf (3.9 mL). After completion of the reaction, triethylamine was added to quench the reaction. The system was concentrated, and the resulting residue was pulped in ethyl acetate, filtered, and concentrated to obtain compound 11 (23.4 g, yield: 66%).
MS m/z (LC-MS): 331.05[M+23]

### Step 5

Compound 11 (26.5 g), 10% Pd/C (7.9 g) and tetrahydrofuran (86 mL) were added to a reaction flask and stirred. A hydrogenation reaction was carried out at 1 atm. After completion of the reaction, the reaction solution was filtered, and purified by column chromatography (eluent: DCM: CH₃OH= 5:1) to obtain compound 12 (15.8 g, yield: 85%).
MS m/z (LC-MS): 219.05[M+1]

### Step 6

Compound 12 (6.04 g), dichloromethane (60 mL) and DMF (0.1 mL) were added to a reaction flask, followed by the addition of 3.5 mL of oxalyl chloride. The reaction solution was heated to 30°C, and reacted for one hour. The reaction solution was concentrated to dryness, and dissolved in 30 mL of dichloromethane for later use.

5.03 g of compound 2, 50 mL of dichloromethane and 11.43 mL of DIEA were added to a reaction flask and stirred, and then the above dichloromethane solution was added. After completion of the reaction, 100 mL of dichloromethane was added. The solution was washed with saturated sodium bicarbonate solution, and dried over anhydrous sodium sulfate. The organic phase was concentrated to obtain compound 13 (9.5 g).
MS m/z (LC-MS): 378.10[M+1]

### Step 7

Compound 13 (9.18 g) and isopropanol (90 mL) were added to a reaction flask, followed by the addition of 6.72 g of potassium carbonate. The reaction solution was heated to 50°C and stirred. After completion of the reaction, the reaction solution was filtered. The filtrate was concentrated to dryness, and 100 mL of methyl *tert-butyl* ether was added to dissolve the residue. The solution was filtered, and the filtrate was concentrated to dryness to obtain compound 14 (7.76 g).
MS m/z (LC-MS): 336.10[M+1]

### Step 8

Compound 14 (7.76 g) and dichloromethane (80 mL) were added to a reaction flask, followed by the addition of 6.43 mL of triethylamine and 7.69 g of p-nitrobenzenesulfonyl chloride. The reaction solution was stirred at room temperature. After completion of the reaction, 100 mL of dichloromethane was added. The solution was washed with saturated sodium bicarbonate solution and saturated ammonium chloride solution successively, and dried over anhydrous sodium sulfate. The reaction system was concentrated, and the residue was pulped in *n*-heptane: dichloromethane= 50:1 (30 mL), filtered, and dried to obtain compound 15 (8.23 g).
MS m/z (LC-MS): 521.00[M+1]

### Example 4

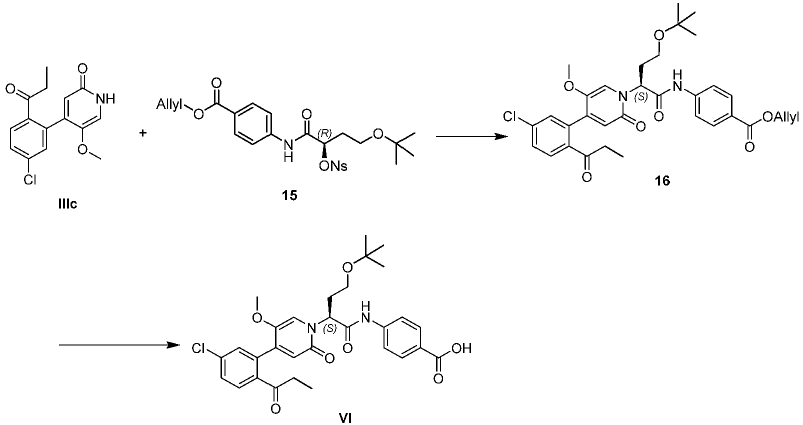

### Step 1

Compound IIIc (50 mg, prepared according to the method disclosed in the patent application WO2018041122) was dissolved in 1 mL of dioxane, followed by the addition of compound 15 (9.5 g, 23.55 mmol) and Cs₂CO₃ powder (168 mg). The reaction solution was reacted at room temperature. After completion of the reaction, the reaction solution was filtered. The filtrate was concentrated to dryness, and the resulting residue was purified by column chromatography (eluent: ethyl acetate: petroleum ether= 1:1) to obtain compound 16 (65 mg, optical purity: 96.5%).

### Step 2

Compound 16 (1 g) was dissolved in 6 mL of tetrahydrofuran, followed by the addition of 0.148 mL of pyrrolidine and 24 mg of Pd(dppf)Cl₂ under stirring. The reaction solution was reacted at room temperature. After completion of the reaction, an appropriate amount of methyl *tert-butyl* ether was added, and the solution was extracted with saturated aqueous sodium carbonate solution. The aqueous phase was washed with methyl *tert-butyl* ether, adjusted to be acidic with hydrochloric acid, and extracted with ethyl acetate. The organic phase was washed with saturated brine, dried over anhydrous sodium sulfate, and concentrated to obtain compound VI (0.84 g, yield: 90.6%, optical purity: 98%).

Since the present disclosure has been described according to specific embodiments, certain modifications and equivalent changes are obvious to those skilled in the art and are included in the scope of the present disclosure.

## Claims

1. A method for preparing a compound of formula AI, comprising a step of reacting a compound of formula III and a compound of formula II, 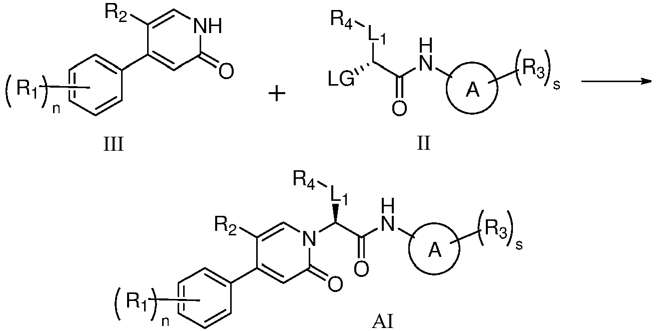 wherein:
ring A is an aryl or heteroaryl;
each R₁ is identical or different and each is independently selected from the group consisting of hydrogen atom, halogen, alkyl, haloalkyl, alkoxy, haloalkoxy, amino, nitro, cyano, hydroxy, hydroxyalkyl, -C(O)R₅, cycloalkyl, heterocyclyl, aryl and heteroaryl;
R₂ is selected from the group consisting of halogen, alkyl, haloalkyl, alkoxy, cycloalkyloxy, haloalkoxy, amino, nitro, cyano, hydroxy, hydroxyalkyl and alkylthio, wherein the alkyl, alkoxy, haloalkyl, haloalkoxy, hydroxyalkyl and alkylthio are each optionally further substituted by one or more substituent(s) selected from the group consisting of deuterium atom, halogen, alkoxy, haloalkoxy, amino, nitro, cyano, hydroxy and hydroxyalkyl;
L₁ is an alkylene, wherein the alkylene is optionally further substituted by one or more substituent(s) selected from the group consisting of halogen and deuterium atom;
each R₃ is identical or different and each is independently selected from the group consisting of hydrogen atom, alkyl, alkoxy, oxo, halogen, haloalkyl, haloalkoxy, amino, nitro, cyano, hydroxy, hydroxyalkyl, cycloalkyl, heterocyclyl, aryl, heteroaryl, -C(O)R₆, -C(O)OR₆, -NHC(O)R₆, -NHC(O)OR₆, -NR₇R₈, -C(O)NR₇R₈, -CH₂NHC(O)OR₆, -CH₂NR₇R₈, -C(O)OCH(R₇)R₈ and -S(O)ₘR₆, wherein the alkyl is optionally further substituted by one or more substituent(s) selected from the group consisting of deuterium atom, halogen, alkoxy, haloalkoxy, amino, nitro, cyano, hydroxy, hydroxyalkyl, -NR₇R₈ and -NHC(O)OR₆;
R₄ is selected from the group consisting of hydrogen atom, deuterium atom, halogen, alkyl, alkoxy, amino, nitro, cyano, hydroxy, hydroxyalkyl, cycloalkyl, cycloalkyloxy, heterocyclyl, aryl and heteroaryl, wherein the cycloalkyl, heterocyclyl, aryl and heteroaryl are each optionally further substituted by one or more substituent(s) selected from the group consisting of deuterium atom, halogen, alkyl, alkoxy, haloalkyl, amino, nitro, cyano, hydroxy, hydroxyalkyl, cycloalkyl, cycloalkyloxy, heterocyclyl, -NHC(O)R₉ and R₁₀;
R₅ is selected from the group consisting of hydrogen atom, alkyl, haloalkyl, alkoxy, haloalkoxy, amino, hydroxy, hydroxyalkyl, cycloalkyl, heterocyclyl, aryl and heteroaryl, wherein the alkyl, alkoxy, cycloalkyl, heterocyclyl, aryl and heteroaryl are each optionally further substituted by one or more substituent(s) selected from the group consisting of hydrogen atom, deuterium atom, halogen, alkyl, alkoxy, haloalkoxy, amino, nitro, cyano, hydroxy, hydroxyalkyl, cycloalkyl, cycloalkyloxy, heterocyclyl, aryl and heteroaryl;
R₆ is selected from the group consisting of hydrogen atom, alkyl, haloalkyl, hydroxy, amino, cycloalkyl, heterocyclyl, aryl, heteroaryl and carboxy protecting group;
R₇ and R₈ are identical or different and are each independently selected from the group consisting of hydrogen atom, alkyl, haloalkyl, cycloalkyl, heterocyclyl, -C(O)OR₆ and -OC(O)OR₉, wherein the cycloalkyl and heterocyclyl are each optionally further substituted by one or more substituent(s) selected from the group consisting of deuterium atom, halogen, alkyl, alkoxy, haloalkyl, oxo, amino, nitro, cyano, hydroxy and hydroxyalkyl;
R₉ is selected from the group consisting of hydrogen atom, alkyl, alkoxy, haloalkyl, haloalkoxy, amino, cycloalkyl, heterocyclyl, aryl and heteroaryl;
R₁₀ is an aryl or heteroaryl, wherein the aryl and heteroaryl are each optionally further substituted by one or more substituent(s) selected from the group consisting of hydrogen atom, deuterium atom, halogen, alkyl, alkoxy, haloalkyl, amino, nitro, cyano, hydroxy, hydroxyalkyl, cycloalkyl, cycloalkyloxy and heterocyclyl;
LG is a leaving group, and preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
n is 0, 1, 2, 3 or 4;
m is 0, 1 or 2; and
s is 0, 1, 2, 3 or 4.

2. The method according to claim 1, **characterized in that** the compound of formula III reacts with the compound of formula II in the presence of an alkaline substance, the alkaline substance is preferably one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)₂, LiORₐ, NaORₐ, KORₐ, CsORₐ, T1ORₐ, NR_{b}R_{c}R_{d}, DBU, DBN, Mg(ORₐ)₂, LiRₑ, NaNH2, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, X is a halogen, and the alkaline substance is more preferably one or more of tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ and Na₃PO₄.

3. The method according to claim 1, **characterized in that** the substituted sulfonyloxy is selected from the group consisting of C₁-C₆ alkylsulfonyloxy, perfluoro C₁-C₆ alkylsulfonyloxy, arylsulfonyloxy, aralkylsulfonyloxy and heteroarylsulfonyloxy.

4. The method according to claim 1, **characterized in that** the compound of formula II is 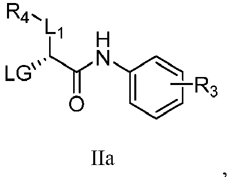 wherein L₁, R₃, R₄ and LG are as defined in claim 1.

5. The method according to claim 1, **characterized in that** the compound of formula II is 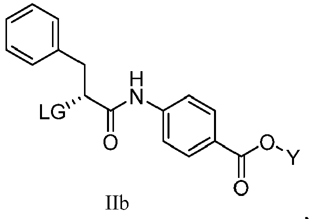 wherein LG is a leaving group, preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS-, substituted or unsubstituted phosphoryloxy or substituted formyloxy; and
Y is selected from the group consisting of hydrogen atom and carboxy protecting group, and preferably a hydrogen atom, methyl, ethyl, allyl, isopentenyl and trimethylsilylethyl.

6. The method according to claim 1, **characterized in that** the compound of formula II is 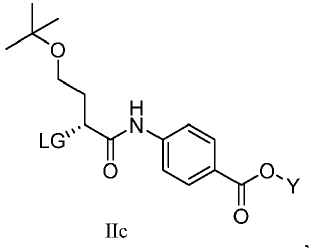 wherein LG is a leaving group, preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS-, substituted or unsubstituted phosphoryloxy or substituted formyloxy; and
Y is selected from the group consisting of hydrogen atom and carboxy protecting group, and preferably a hydrogen atom, methyl, ethyl, allyl, isopentenyl and trimethylsilylethyl.

7. The method according to claim 1, **characterized in that** the compound of formula III is 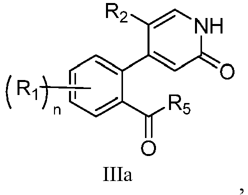 wherein each R₁ is identical or different and each is independently selected from the group consisting of hydrogen atom, halogen, alkyl, haloalkyl, alkoxy, haloalkoxy, amino, nitro, cyano, hydroxy, hydroxyalkyl, cycloalkyl, heterocyclyl, aryl and heteroaryl;
R₅ is selected from the group consisting of alkyl, cycloalkyl and haloalkyl, wherein the alkyl and cycloalkyl are each optionally further substituted by one or more substituent(s) selected from the group consisting of deuterium atom, halogen, alkyl and cycloalkyl; and
R₂ and n are as defined in claim 1.

8. The method according to claim 1, **characterized in that** the compound of formula III is

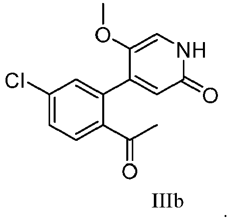

9. The method according to claim 1, **characterized in that** the compound of formula III is

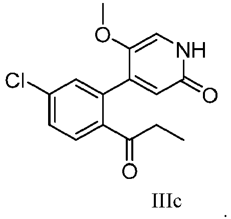

10. The method according to claim 1, **characterized in that** the method also comprises a step of reacting a compound of formula IV and a compound of formula V to obtain the compound of formula II, 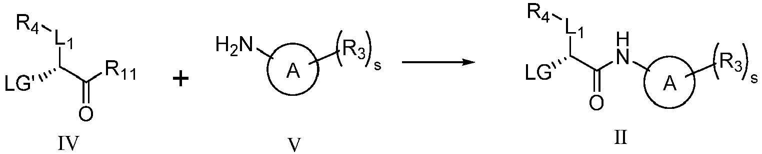 wherein R₁₁ is selected from the group consisting of -OR₉ and halogen;
LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and
L₁, R₃, R₄, R₉, LG and s are as defined in claim 1.

11. A compound of formula lib, 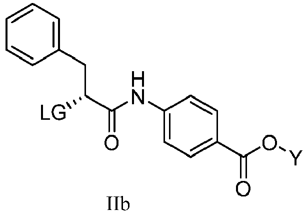 wherein LG is a leaving group, preferably a halogen, substituted sulfonyloxy, RᵢRⱼN-, hydroxy, RₖS- or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS- or substituted formyloxy; and
Y is selected from the group consisting of hydrogen atom and carboxy protecting group, and preferably a hydrogen atom, methyl, ethyl, allyl, isopentenyl and trimethylsilylethyl; and
and when LG is a hydroxy, Y is not a methyl.

12. A compound of formula IIc, 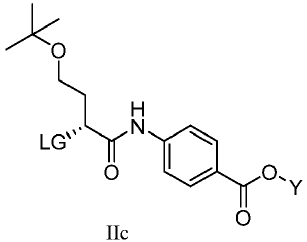 wherein LG is a leaving group, preferably a halogen, substituted sulfonyloxy, RᵢRⱼN-, hydroxy, RₖS-, substituted or unsubstituted phosphoryloxy or substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl;
and more preferably a halogen, C₁-C₆ alkylsulfonyloxy, perfluoro C₁-C₆ alkylsulfonyloxy, arylsulfonyloxy, aralkylsulfonyloxy, heteroarylsulfonyloxy, RᵢRⱼN-, hydroxy, RₖS- or substituted formyloxy; and
Y is selected from the group consisting of hydrogen atom and carboxy protecting group, and preferably a hydrogen atom, methyl, ethyl, allyl, isopentenyl and trimethylsilylethyl.

13. A method for preparing the compound of formula IIb according to claim 11, comprising a step of reacting a compound of formula IVb or a salt thereof and a compound of formula Vb or a salt thereof, 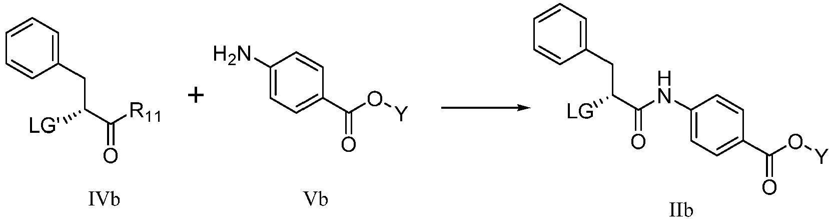 wherein R₁₁ is selected from the group consisting of -OR₉ and halogen;
R₉ is selected from the group consisting of hydrogen atom, alkyl, alkoxy, haloalkyl, haloalkoxy, amino, cycloalkyl, heterocyclyl, aryl and heteroaryl;
LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and
LG and Y are as defined in claim 11.

14. A method for preparing the compound of formula IIc according to claim 12, comprising a step of reacting a compound of formula IVc or a salt thereof and a compound of formula Vb or a salt thereof, 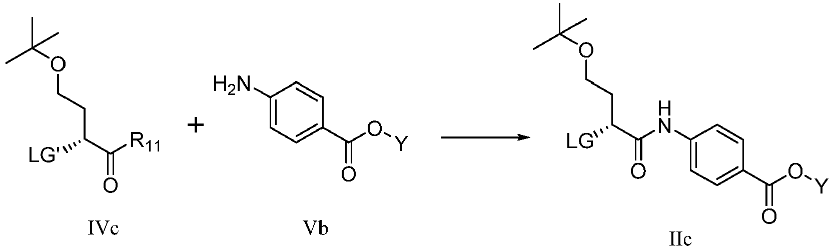 wherein R₁₁ is selected from the group consisting of -OR₉ and halogen;
R₉ is selected from the group consisting of hydrogen atom, alkyl, alkoxy, haloalkyl, haloalkoxy, amino, cycloalkyl, heterocyclyl, aryl and heteroaryl;
LG' and LG are identical or different and are each selected from the group consisting of halogen, substituted sulfonyloxy, RᵢRⱼN-, hydroxy, RₖS-, substituted or unsubstituted phosphoryloxy and substituted formyloxy, wherein Rᵢ and Rⱼ are each independently selected from the group consisting of hydrogen atom, C₁-C₆ alkyl and amino protecting group, and Rₖ is selected from the group consisting of hydrogen atom and C₁-C₆ alkyl; and
LG and Y are as defined in claim 12.

15. A method for preparing a compound of formula I or the pharmaceutically acceptable salt thereof, comprising a step of reacting the compound of formula IIIb and the compound of formula IIb to obtain a compound of formula BI, wherein LG and Y are as defined in claim 5,

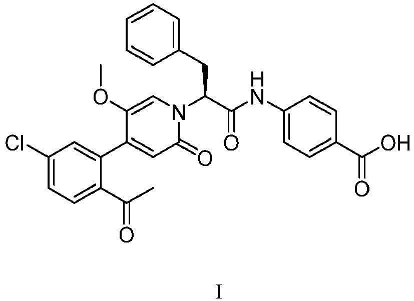

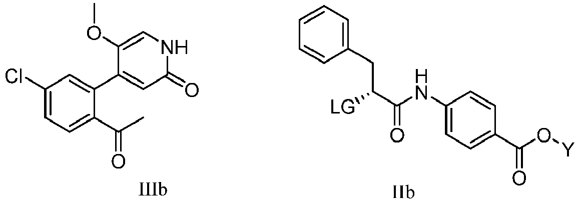

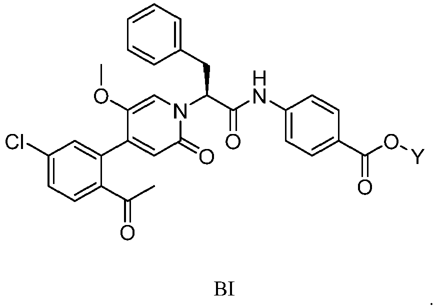

16. The method according to claim 15, **characterized in that** the compound of formula IIIb reacts with the compound of formula IIb in the presence of an alkaline substance, the alkaline substance is preferably one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)2, LiORₐ, NaORₐ, KORₐ, CsORₐ, T1ORₐ, NR_{b}R_{c}R_{d}, DBU, DBN, Mg(ORₐ)₂, LiRₑ, NaNH₂, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, X is a halogen, and the alkaline substance is more preferably one or more of tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ and Na₃PO₄.

17. The method according to claim 15, **characterized in that** the method also comprises the step for preparing the compound of formula IIb according to claim 13.

18. The method according to claim 15, **characterized in that** LG is a substituted sulfonyloxy, and the method also comprises a step of converting a compound of formula lid into the compound of formula IIb,

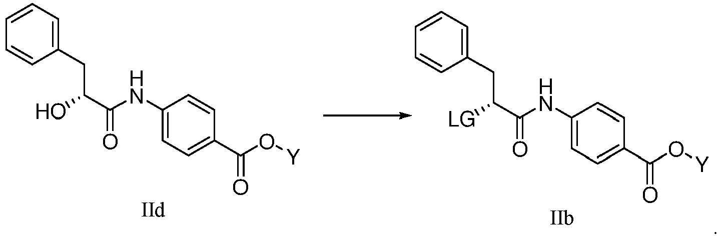

19. The method according to claim 15, **characterized in that** Y is a carboxy protecting group, and the method also comprises a step of removing the carboxy protecting group of the compound of formula BI.

20. A method for preparing a compound of formula VI or the pharmaceutically acceptable salt thereof, comprising a step of reacting the compound of formula IIIc and the compound of formula IIc to obtain a compound of formula BVI, wherein LG and Y are as defined in claim 5,

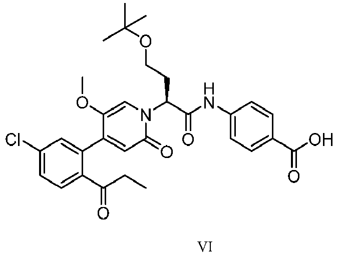

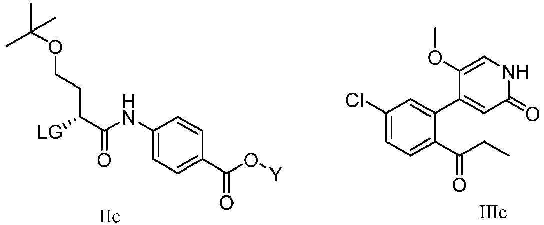

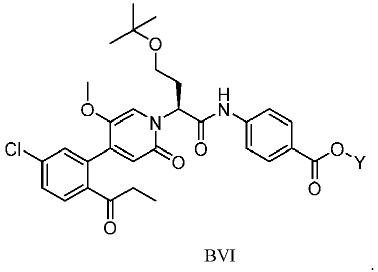

21. The method according to claim 20, **characterized in that** the compound of formula IIIc reacts with the compound of formula IIc in the presence of an alkaline substance, the alkaline substance is preferably one or more of Na₂CO₃, K₂CO₃, Cs₂CO₃, NaHCO₃, KHCO₃, Na₂HPO₄, K₂HPO₄, K₃PO₄, Na₃PO₄, LiHMDS, NaHMDS, KHMDS, Ba(OH)2, LiORₐ, NaORₐ, KORₐ, CsORₐ, T1ORₐ, NR_{b}R_{c}R_{d}, DBU, DBN, Mg(ORₐ)₂, LiRₑ, NaNH₂, KNH₂, LiNH₂, LiH, NaH, KH, CaH₂, MgH₂, R_{f}MgX, KF, CsF, Bu₄F and quaternary ammonium salt, wherein each Rₐ is independently selected from the group consisting of hydrogen atom and C₁-C₆ alkyl, R_{b}, R_{c} and R_{d} are each independently selected from the group consisting of hydrogen atom, alkyl and cycloalkyl, Rₑ is selected from the group consisting of iBu, nBu, tBu, cyclohexyl, phenyl and 2,2,6,6-tetramethylpiperidinyl, R_{f} is a C₁-C₆ alkyl, X is a halogen, and the alkaline substance is more preferably one or more of tBuOK, LiHMDS, NaHMDS, KHMDS, LiH, NaH, KH, CaH₂, MgH₂, TBAI, TBAF, TBAB, TBAC, Na₂CO₃, K₂CO₃, Cs₂CO₃, K₃PO₄ and Na₃PO₄.

22. The method according to claim 20, **characterized in that** the method also comprises the step for preparing the compound of formula IIc according to claim 14.

23. The method according to claim 20, **characterized in that** LG is a substituted sulfonyloxy, and the method also comprises a step of converting a compound of formula IIe into the compound of formula IIc,

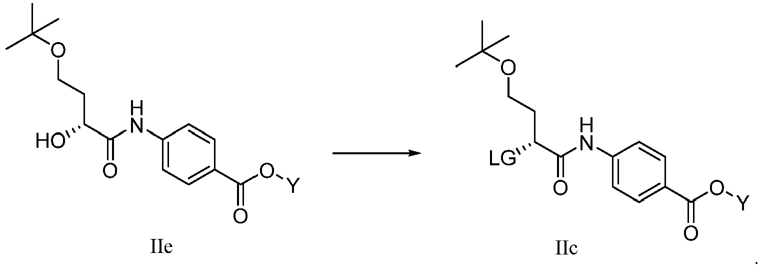

24. The method according to claim 20, **characterized in that** Y is a carboxy protecting group, and the method also comprises a step of removing the carboxy protecting group of the compound of formula BVI.

25. A compound of formula IVd, 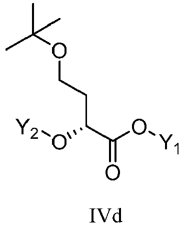 wherein Y1 is selected from the group consisting of hydrogen atom and carboxy protecting group; and Y2 is selected from the group consisting of hydrogen atom and hydroxy protecting group.

26. A method for preparing the compound of formula IVd according to claim 25, comprising a step of converting a compound of formula IVe into the compound of formula IVd,
